(19)

Europäisches
Patentamt
European
Patent Office
Office européen
des brevets

(11) **EP 2 348 246 A1**

(12) **EUROPEAN PATENT APPLICATION**
published in accordance with Art. 153(4) EPC

(43) Date of publication:
**27.07.2011 Bulletin 2011/30**

(21) Application number: **09819251.1**

(22) Date of filing: **08.10.2009**

(51) Int Cl.:
*F21S 2/00* (2006.01)　　*A61M 21/02* (2006.01)
*H01L 51/50* (2006.01)　　*F21Y 101/02* (2006.01)
*F21Y 105/00* (2006.01)　　*H01L 33/00* (2010.01)

(86) International application number:
**PCT/JP2009/067573**

(87) International publication number:
**WO 2010/041717 (15.04.2010 Gazette 2010/15)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(30) Priority: **08.10.2008 JP 2008262194**

(71) Applicant: **Kabushiki Kaisha Hayashibara
Seibutsu
Kagaku Kenkyujo
Kita-ku
Okayama-shi
Okayama 700-0907 (JP)**

(72) Inventors:
• **MIYAKE Toshio**
**Okayama-shi**
**Okayama 700-0907 (JP)**
• **FUKUDA Shigeharu**
**Okayama-shi**
**Okayama 700-0907 (JP)**

(74) Representative: **Daniels, Jeffrey Nicholas
Page White & Farrer
Bedford House
John Street
London WC1N 2BF (GB)**

(54) **LIGHTING DEVICE**

(57)　　The aims of the present invention are to provide a lighting device, which can be used as illumination for ordinary life space, and has the functions of activating human serotonin nervous system, substantially not raising noradrenaline level in blood, decreasing aggressiveness, and reducing the factors that induce so-called "kireru" state. The aims are attained by providing a lighting device which radiates light containing near ultraviolet radiation having a wavelength in the range of 320 nm to 380 nm along with visible light.

EP 2 348 246 A1

**Description**

Field of the Invention

The present invention relates to a lighting device, in particular, a lighting device which radiates light having the functions of activating human serotonin nervous system, substantially not raising noradrenaline level in human blood, and suppressing aggressiveness to decrease the factors that may invite a state of so-called "kireru" (i.e. a state of getting excited and losing one's reason).

Background of the Invention

In the complicated modern society, there are growing many kinds of stresses, such as mental stresses caused by increased tension, suppression, anxiety, discontent, anger, or irritation which may be encountered, due to the change of social structure, through human relationship formed in workplaces or communities , stresses caused by environmental deterioration including air pollution and water pollution, and stresses suffered from electromagnetic waves emitted by mobile phones, various information displaying apparatuses, various means of transportation, and so on. In addition, many people recently tend to work until midnight as the people's active time shifts from daytime to nighttime, and works which are done from midnight to daybreak are increasing in various fields and in various manner. In such changes of social conditions, the number of persons who become impulsive and take aggressive action at trivial matters, namely turn the state of so-called "kireru", has been increasing and some of them sometimes may end in indiscriminate murder, which is a big social problem. As there are anxieties about the increase of mental pain due to the change of social structure and the increase of crimes caused thereby, it is desired to establish living environment where the factors inducing mentally unstable state called "kireru" are decreased.

It is said that serotonin nervous system is so greatly involved in aggressive behavior of animals including human beings that the fall of serotonin level in brain caused by inactivation of serotonin nervous system may induce emotionally unstable state and aggressive behavior (See "Brain deficient in serotonin" by Hideho ARITA, NHK SHUPPAN, 2003, for example). It is said that taking natural sunshine is one of the effective ways to activate serotonin nervous system to increase serotonin level in brain. However, natural sunshine is so strong that illuminance of 2500 lux or more is needed to obtain the same effect as the sunshine by artificial light, as in the case of phototherapy for treating winter depression. Since the illuminance of illuminations at nighttime or in rooms in ordinary living space such as business offices and homes is in the range of 200 to 1000 lux, the lighting devices used for above mentioned phototherapy are not suitable for the use as illumination of ordinary living space. On the other hand, it has been said from long time ago that feelings and moods of human beings are greatly affected by colors. Light of blue color is effective to calm mental state down, and it is said that this is because light comprising a lot of blue light components has a function of inducing secretion of serotonin. While lighting devices radiating blue light are increasingly employed for streetlights etc., for example, in order to decrease crimes utilizing the effects of blue light as above mentioned, it is white light in the nature of light bulb color or daylight color (color temperature is in the range of about 2700 to 6500) that is used for the illumination of ordinary living space, and blue light is not suitable because of its color rendering property.

It is known that the level in blood of noradrenaline, one of neurotransmitters, increases when aggressiveness becomes stronger, and noradorenaline is considered to be one of neurotransmitters which are involved in aggressiveness (See, for example, Randy J. Nelson and Brian C. Trainor, "Neural mechanism of aggression", Nature reviews/Neuroscience, Viol.8, pp.536-546 (2007)).

A light device, however, light of which is white light being used for lighting ordinary living space, activates human serotonin nervous system even at such illuminance level as being able to be used for the ordinary illumination, not increases noradrenaline level in blood, and suppressing aggressiveness to decrease the factors that induce the state of "kireru", has not been known.

Disclosure of the Invention

Under the circumstance as mentioned above, the aim of the present invention is to provide a lighting device, which exhibits the functions of activating human serotonin nervous system and not increasing noradrenaline level in blood, and is able to decrease the factors that induce a state in which trivial matters in living environment cause emotional and aggressive behavior, i.e. a state so-called "kireru", through the use as an ordinary lighting device at nighttime or in rooms.

The inventors of the present invention have earnestly studied the relationship between the functions of suppressing the factors that induce the state of "kireru" and light, and found unexpectedly that light comprising near ultraviolet radiation having a wavelength of 320 nm or longer but shorter than 380 nm along with visible light having a wavelength of 380 nm or longer but not longer than 780 nm (called "visible light" hereinafter in the specification) has a function of activating human serotonin nervous system, and further has a function of substantially not increasing noradrenaline level in blood. The inventors have further found that a lighting device using a light source that radiates said light, when used as an ordinary lighting device at nighttime or in rooms, activates human serotonin nervous system, substantially not increases noradrenaline level in blood, and decreasing the factors that induce human beings to the state "kireru" to accomplish the present invention.

The present invention attains the aims by providing a lighting device, which radiates light comprising near ultraviolet radiation having a wavelength of 320 nm or longer but shorter than 380 nm along with visible light.

A lighting device of the present invention, through the use as an ordinary lighting device, is able to activate human serotonin nervous system, substantially not increase noradrenaline level in blood, and suppress aggressiveness so as to inhibit human beings from falling into the state of "kireru".

Simple explanations of drawings

Figure 1 is a block diagram of a lighting device Embodiment 1 according to the present invention.
Figure 2 is an emission spectrum of the light radiated by a lighting device Embodiment 1 according to the present invention.
Figure 3 is a block diagram of a lighting device Embodiment 2 according to the present invention.

Explanations of symbols

| | |
|---|---|
| $LD1_{11}$ - $LD1_{mn}$ | LED radiating visible light |
| $LD2_{11}$ - $LD2_{ij}$ | LED radiating near ultraviolet radiation |
| 3 | Commercial AC power source |
| 4, 5 | Power source circuit |
| 6, 7 | Lightning circuit |
| 8 | Control circuit |
| $ED9_{11}$ - $ED9_{mn}$ | Organic EL radiating visible light |
| $LD10_{11}$ - $LD10_{ij}$ | LED radiating near ultraviolet radiation |
| 11, 12 | Power source circuit |
| 13, 14 | Lightning circuit |
| 15 | Control circuit |

Best Mode for Carrying out the Invention

Activation of serotonin nervous system referred to in the present invention means that serotonin level in brain rises to maintain the state mentally stable. Since serotonin level in brain has positive correlation with serotonin concentration in blood or urine according to Hideho ARITA, "Brain deficient in serotonin", NHK SHUPPAN, 2003, it is possible to decide if serotonin level in brain rises to activate serotonin nervous system by measuring serotonin concentration in blood or urine.

Not rising noradrenaline level in blood referred to in the present invention means that, when a work which causes mental load is conducted under a light, noradrenaline concentration in blood does not substantially change before and after the work.

With regard to the composition of a lighting device according to the present invention, a lighting device of the present invention comprises a light source which radiates light activating human serotonin nervous system and not rising noradrenaline level in blood when used as ordinary illumination at nighttime or in rooms, a power source supplying electric energy to the light source, and a control device which controls radiation intensity of the light emitted by the light source.

There are no limitations concerning the light source, the power source and the control device which are used in the lighting device of the present invention so long as the lighting device of the present invention radiate light which comprises near ultraviolet radiation with a wavelength of 320 nm or longer but shorter than 380 nm along with visible light, and said light activates human serotonin nervous system and not rises noradrenaline level in blood when the lighting device is used as ordinary illumination at nighttime or in rooms. The light radiated from a lighting device of the present invention preferably comprises near ultraviolet radiation with a wavelength of 320 nm or longer but shorter than 380 nm in 3 to 15%, more preferably 5 to 10% in terms of radiant energy (irradiance (W/cm$^2$/nm)) of the visible light.

When the radiant energy of the near ultraviolet radiation with a wavelength of 320 nm or longer but shorter than 380 nm is less than 3% of that of the visible light, advantageous effects of the present invention would not be obtained. On the other hand, when the radiant energy of the near ultraviolet radiation goes over 15% of that of the visible light, the advantageous effects of the present invention would not be intensified by the increased near ultraviolet radiation components, and skin and other things sensitive to ultraviolet radiation may be affected by long time ultraviolet radiation. In addition, it is preferable that the near ultraviolet radiation radiated by a lighting device of the present invention does not comprise light having a wavelength of less than 300 nm, which may give relatively strong influence on living bodies. Since a lighting device of the present invention is to be used as ordinary illumination at nighttime or in rooms, it is preferable that the lighting device of the present invention radiates white light in the nature of light bulb color or daylight color, more preferably, of 3500 to 5000K, gives illuminance of 200 lux or more, more preferably 500 to 1000 lux, at irradiated surface, and exhibits high color rendering property with general color rendering index (Ra) of 90 or more, more preferably 95 or more.

Light emitting diode (LED), organic electroluminescent device (organic EL), inorganic electroluminescent device (inorganic EL), incandescent lamp, fluorescent lamp, electrodeless fluorescent lamp, discharge lamp, and so on can be utilized as a light source used in a lighting device of the present invention. Among the light sources, LED and organic EL are particularly superior as a light source of the present invention because they have various features such as high luminous efficacy and low electricity consumption, long life time, being easy to be handled due to their small size, containing no hazardous substances such as mercury, emitting less infrared and being suitable for forming a light source with suppressed heat generation, low electricity consumption, and so on. An LED light source, in which semiconductor light emitting device radiating blue light is used as a light source for excitation, and various fluorescent materials emitting fluorescence in red or green color respectively are combined to emit white light, is preferably used as an LED that emits light of visible light component, because the light emitted from such LED light source contains lights in three primary colors and reveals high color rendering property. Furthermore, an LED light source, in which semiconductor light emitting device radiating light in the range of near ultraviolet to violet radiation is used as a light source for excitation, and various fluorescent materials emitting fluorescence in red, green, or blue color respectively when excited by the light source for excitation are combined to emit white light, is particularly preferable because it can emit near ultraviolet radiation suitable for a lighting device of the present invention and visible light at the same time, as well as the lights emitted by the LED light source exhibit high color rendering property.

It doesn't matter for the light source of the present invention whether it uses a light source emitting visible light in combination with a light source emitting near ultraviolet radiation having a wavelength of 320 nm or longer but shorter than 380 nm, or it uses a single light source emitting light that comprises visible light along with near ultraviolet radiation having a wavelength of 320 nm or longer but shorter than 380 nm, so long as the advantageous effects of the present invention are obtained. When a light source emitting visible light and a light source emitting near ultraviolet radiation having a wavelength of 320 nm or longer but shorter than 380 nm are used in combination, it is preferable to place both light sources so that the lights from the both light sources are mixed. It is also preferable to use diffusive screen, reflector, lens, etc. so that uniform light without unevenness in color or luminance is obtained at irradiated surface.

When a light source which radiates light containing ultraviolet components relatively abundantly is used, it is preferable to use suitable ultraviolet-absorbing filter so as to remove harmful ultraviolet components in the wavelength region of affecting eyes or skin badly to obtain light comprising near ultraviolet radiation having a wavelength in the range of 320 to 380 nm in an amount of 3 to 15% in terms of the radiant energy of the visible light. Furthermore, when a light source which radiates light that contains ultraviolet components less than visible components, but is relatively abundant in quantity of light is used, it is possible to use suitable filter to reduce visible light components in order to obtain light containing near ultraviolet radiation having a wavelength in the range of 320 to 380 nm in an amount of 3 to 15% in terms of the radiant energy of the visible light and to use the light source in a lighting device of the present invention.

Form and appearance of the lighting device of the present invention can be varied according to its use. That is, when the lighting device of the present invention is used to illuminate inside or outside of houses such as houses for individual, condominiums, apartments and housing developments; and various constructions or structures such as libraries, schools, studios, beauty shops, hospitals, factories, office buildings, offices, Japanese-style inns, hotels, restaurants, banquet rooms, halls for wedding ceremony, convention halls, stores, supermarkets, department stores, art museums, museums, concert halls, halls, aircrafts, vehicles, gymnasiums, stadiums, livestock barns, henhouses, fish farms, animal factories and plant factories, a light source as mentioned above and a power source for supplying electric power to the light source are placed in or on desktop lighting devices such as adjustable lamps, desk lamps, hurricane lamps, table lamps and mini-lamps, or lighting apparatuses for indoor or outdoor such as under shelf lamps, ceiling lamps, downlights, wall lamps, pendent lamps, chandeliers, swag lamps, floor lamps, garden lamps, gate lamps, and said lighting devices or lighting apparatuses are set or installed at appropriate places inside or outside of studies, ateliers, child rooms, bed rooms, living rooms, dining rooms, kitchens, toilets, washrooms, bath rooms, corridors, stairs, balconies, entrance rooms, reading rooms, classrooms, halls, lobbies, waiting rooms, treatment rooms, operating rooms, control rooms, offices, drawing rooms, laboratories, lounges, guest rooms, clerk rooms, cooking rooms, driver's rooms, breeding rooms, and cultivating rooms in houses or institutions.

Working examples of a lighting device of the present invention are explained below raising embodiments for example. However, the present invention is not limited to the embodiments below mentioned.

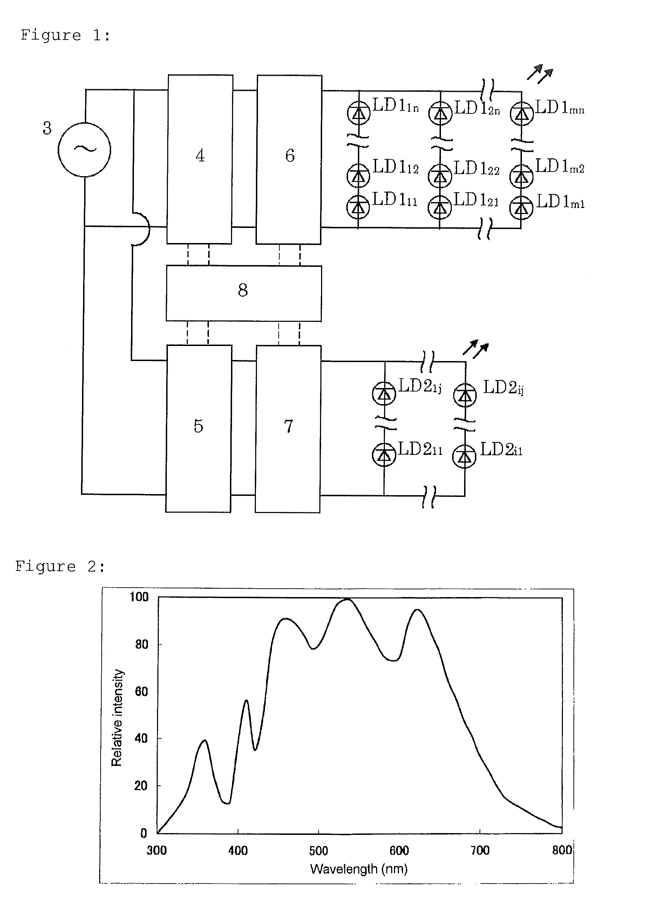

Figure 1 shows Embodiment 1 of the lighting device of the present invention, which uses LED for lighting sources and is the most basic block diagram. In Figure 1, $LD1_{11}$ - $LD1_{mn}$ are LED which radiate white visible light (color temperature of about 4000 K), and $LD2_{11}$ - $LD2_{ij}$ are LED which radiate near ultraviolet radiation having a peak at the wavelength near 370 nm. LD1 and LD2 connected in series, in parallel, or in series-parallel, respectively, can be used so long as the radiant energy of near ultraviolet radiation having a wavelength in the range of 320 to 380 nm is in the range of 3 to 15% of that of visible light in the light emitted by the lighting device. The number of LD1 and LD2 can be decided arbitrary. 3 is an AC power source for commercial use. 4 and 5 are power source circuits which obtain DC voltages supplied to the LED radiating visible light (LD1) and the LED radiating near ultraviolet radiation (LD2), respectively, from AC power source 3. 6 and 7 are lightning circuits which lighten the LED radiating visible light (LD1) and the LED radiating near ultraviolet radiation (LD2), respectively. Lightning circuits 6 and 7 are controlled by control circuit 8 so that the radiant energy of near ultraviolet radiation having a wavelength in the range of 320 to 380 nm is in the range of 3 to 15%, preferably in the range of 5 to 10%, of that of visible light in the light emitted by the lighting device. Figure 2 shows an example of an emission spectrum of the lighting device. The peak near the wavelength of 370 nm corresponds to LD2, and the peaks near the wavelengths of 420 nm, 460 nm, 530 nm and 620 nm correspond to LD1. Any LED, including suitable LED radiating near ultraviolet radiation available on the market, can be used as LD2 so long as the advantageous effects of the present invention are obtained. While any LED can be used as LD1 so long as the advantageous effects of the present invention are obtained, it is preferable from the viewpoint of color rendering property to use an LED in which semiconductor light emitting device radiating light in the range of near ultraviolet to violet radiation is used as a light source for excitation, and suitable fluorescent materials emitting fluorescence in red, green, or blue color, respectively, when excited by the light source for excitation are combined to emit white light. For example, when an LED light source which is prepared to emit white light by using a fluorescent material which mainly comprises a fluorescent material having the composition of $(Sr, Ca)AlSiN_3:Eu$ as red one, a fluorescent material having the composition of $BaMgEuAl_{10}O_{17}$ as blue one and a fluorescent material having the composition of $(Ba, Sr)SiO_4:Eu$ as green one, and by using a semiconductor light emitting device in GaN series which has a luminous layer consisting of a material of InGaN family is used as LD1, general color rendering index (Ra) of over 95 can be achieved in the lighting device of the present invention. Further, when said LED light source used as LD1 emits near ultraviolet to violet radiation with a wavelength in the range of 320 nm to 380 nm as well, the relative amount of LD2 in relation to LD1 used in the lighting device can be reduce, and moreover the light source of the lighting device can be composed LD1 only. Although there are no limitations with respect to the forms of the power source circuits, lightning circuits and control circuits so long as the advantageous effects of the present invention are attained, constant-current lighting system, duty-controlled lighting system, and dynamic lighting system are preferably employed because of their high lightning efficiency and small fluctuation in brightness.

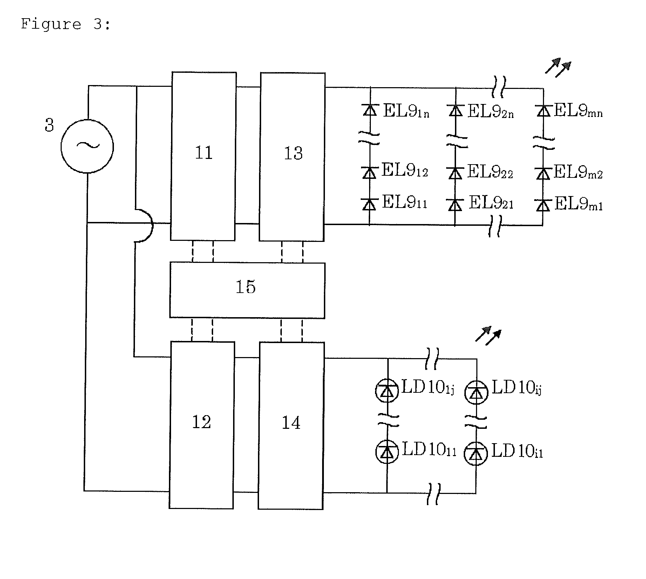

Figure 3 shows block diagram of Embodiment 2 of the lighting device according to the present invention. In Embodiment 2, an organic EL panel in which organic EL devices emitting red, blue or green light are combined to emit white light is used as a light source, and LED is used as a light source emitting light in near ultraviolet radiation region. In Figure 3, $EL9_{11}$ - $EL9_{mn}$ are white light organic EL utilizing pyran derivatives, and $LD10_{11}$ - $LD10_{ij}$ are LED radiating near ultraviolet radiation having a peak at the wavelength of near 375 nm. EL9 and LD10 connected in series, in parallel, or in series-parallel, respectively, can be used so long as the radiant energy of near ultraviolet radiation having a wavelength in the range of 320 to 380 nm is in the range of 3 to 15% of that of visible light in the light emitted by the lighting device. The number of EL9 and LD10 can be decided arbitrary. 11 and 12 are power source circuits which obtain DC voltages supplied to the white light organic EL (EL9) and the LED radiating near ultraviolet radiation (LD10), respectively, from AC power source 3. 13 and 14 are lightning circuits which lighten the white light organic EL (EL9) and the LED radiating near ultraviolet radiation (LD10), respectively. Lightning circuits 13 and 14 are controlled by control circuit 15 so that the radiant energy of near ultraviolet radiation having a wavelength in the range of 320 to 380 nm is in the range of 3 to 15% of that of visible light in the light emitted by the lighting device. The lighting device of this embodiment emits light of neutral color with color temperature of about 5500K, having emission maximums at the wavelengths of near 460 nm, 500 nm and 595 nm, respectively, in visible light region, having quite good color purity showing chromatic coordinate x in the range of 0.3 to 0.35 and y in the range of 0.3 to 0.4 on xy-chromaticity diagram of CIE, and being superior in color rendering property. In addition to pyran derivatives, benzopyran derivatives, coumarin derivatives and cyanocoumarin derivatives are preferably used as the illuminant employed in an organic EL device used in the lighting device as a light source (for example Japanese Patent Kokai 2005-247976).

The lighting device of the present invention is further explained in detail below based on working examples.

(Working Example 1)

<Experiment 1: Influence of the lighting device on human serotonin nervous system>

To investigate the influence of the lighting device of the present invention on human serotonin nervous system, panel test was conducted by sixty adult men (in 20 to 50 years old). Persons tested were randomly divided into six groups with 10 persons each. To the four groups (test sections 1 to 4), deskwork such as reading more than two hours per day under the nighttime illumination produced by using the lighting devices of Embodiment 1 as stand lamp was assigned for one week. The radiant energy of near ultraviolet radiation having a wavelength in the range of 320 to 380 nm emitted by the lighting devices used for the four groups were adjusted to 3, 5, 10, and 15%, respectively. Illuminance at the desk surface was also adjusted to 700 lux for all of the four groups. To one of the remaining two groups, the same deskwork was assigned on the same conditions except that the LEDs emitting the near ultraviolet radiation were not lightened (control section 1). To the other of the remaining two groups, the same deskwork was assigned on the same conditions except that fluorescent lamps of 15 W with worm-white color (color temperature of about 3500K) were lightened at the rated voltage of 100 V to illuminate the desk surface in place of the lighting devices of Embodiment 1 (control section 2). In the light emitted by the fluorescent lamps used in control section 2, the radiant energy of near ultraviolet radiation was about 1% of that of visible light Urine samples were taken from each tested persons before the test and on the last day of the tested period, and preserved at minus 20°C with the addition of 6M hydrochloric acid. After having been defrosted the urine samples, the concentration of serotonin in the urine samples were measured using commercially available serotonin measuring reagent "Serotonin EIA" (prepared by Labor Diagnostika Nord, corporation). Increasing rate of serotonin concentration after the test in relation to the serotonin concentration before the test was calculated by using the following equation. The calculated increasing rates were averaged in each group and shown in Table 1.

$$\text{Increasing rate (\%)} = \{(\text{serotonin concentration after the test}) - (\text{serotonin concentration before the test}) / \text{serotonin concentration before the test}\} \times 100$$

[Table 1]

|  | Light source | Radiant energy of near ultraviolet radiation to that of visible light (%) | Increasing rate of serotonin in urine (%) |
|---|---|---|---|
| Control section 1 | LED | 0 | 3.2 |
| Control section 2 | Fluorescent lamp | 1 | 1.9 |
| Test section 1 | LED | 3 | 20.2 |
| Test section 2 | | 5 | 32.8 |
| Test section 3 | | 10 | 40.6 |
| Test section 4 | | 15 | 38.3 |

As evident from the results shown in Table 1, in the tested persons who passed under the LED light source which contains little near ultraviolet radiation and the fluorescent lamp (control sections 1 and 2), increasing rates of serotonin in urine were less than 5% and serotonin concentrations in urine were almost same before and after the test. Contrary to this, in the tested persons who passed under the illumination containing the near ultraviolet radiation in an amount of 3% or more of the visible light in terms of the radiant energy (test sections 1 to 4), serotonin concentrations in urine increased in an amount of about 20% or more as compared with that of before the test, and increasing rate of serotonin in urine increases as the intensity of the near ultraviolet radiation in relation to the visible light increases. Increasing rate of serotonin in urine became 30 to 40% when the intensity of the near ultraviolet radiation in relation to the visible light was 5% or more. The results above mentioned indicates that human serotonin nervous system is activated by the radiation of light which contains near ultraviolet radiation in an amount of 3 to 15%, more preferably 5 to 10%, in relation to the radiant energy of visible light. From the present experiment, it has become apparent that serotonin nervous system is activated and the factors which induce the so-called "kireru" state are reduced by the light device of the present invention.

(Working Example 2)

<Experiment 2: Influence of the illumination of the present invention on noradrenaline level>

Panel test was conducted by twenty adult men (in 20 to 50 years old). Persons tested were randomly divided into two groups with 10 persons each. To the one group, simple adding work based on Kraepelin test for one hour was assigned (test section) as working load under the illumination produced by using as stand lamp the lighting devices used in Experiment 1 in which the radiant energy of the near ultraviolet radiation was adjusted to 5% in relation to the visible light, and under the circumstance of metronome sounds. Before and after the working load, venous blood was taken and noradrenaline level in blood was measured. To the remaining group, the same work was assigned under the same condition except that the lighting devices used in Experiment 1 in which the near ultraviolet LEDs were not lightened, were used as illumination (control section). The measurement of the noradrenaline level in blood was conducted in SRL Co., Limited. Measured values were averaged and shown in Table 2.

[Table 2]

|  | Noradrenaline (pg/ml) | | Increasing rate (%) |
| --- | --- | --- | --- |
|  | before the work | after the work | |
| Control section | 483.5 | 534.3 | 10.5 |
| Test section | 505.2 | 509.2 | 0.8 |

In control section, noradrenaline level in blood rose after the work load in nine persons tested among ten, and the increasing rate was 10% or more in average as compared with the noradrenaline level before the work load. On the other hand, in test section, noradrenaline level in blood did not changed substantially before and after the work load. From the results above mentioned, it is understood that noradrenaline level in blood of a person who is charged with work load does not substantially rise when the lighting device of the present invention which radiates light containing near ultraviolet radiation components in the range of 320 nm to 380 nm is used. While seven persons tested in control section felt eye tiredness after the Kraepelin test, no one felt eye tiredness in test section. It is considered that the lighting device of the present invention has advantageous effects of preventing eyestrain as well.

(Working Example 3)

<Experiment 3: Influence of the lighting device on mouse aggressiveness>

Thirty BALB/C male mice (seven-weeks old) were divided into three groups with ten mice each and were bred for one week separately with no restrictions on taking powdered feed and water under the circumstance where bright period and dark period repeated in twelve hours each. In one of the three groups, the lighting device of Embodiment 1 in which the radiant energy of the near ultraviolet radiation was adjusted to 10% in relation to the visible light was used for the illumination in the bright period and lightening nothing in the dark period to breed the mice (test section). In the remaining two groups, the same illumination as in test section was used to breed the mice except that light source emitting the near ultraviolet radiation in the lighting device was not lightened, and besides, in one of the two groups, no lamp was lightened in the dark period (control section 1) and in the other of the two groups, only the light source emitting the near ultraviolet radiation was lightened in the dark period at the same intensity as in test section (control section 2). Illuminace at the cages floor was adjusted to about 500 lux in the bright period.

After one week breeding, aggressiveness was observed on each mouse according to the following ways. Each mouse was put in a transparent plastic container to oppose one to one to another mouse of the same weeks old and normally bred with no restrictions on taking powdered feed and water, and the number of times of biting action and threatening action taken in five minutes were counted as aggressive actions. Aggressiveness in test section and control section 2 was obtained in the relative value to the aggressiveness in control section 1 when the number of aggressive actions in control section 1 was set to 100. The average of the aggressiveness in each groups are shown in Table 3. After having observed aggressiveness, frontal lobe cortex was extracted from the brain of each mouse, homogenated in a buffer containing 0.1 % ascorbic acid, and preserved at minus 20°C. After having been defrosted the homogenated sample, the serotonin concentrations in the brain were measured using commercially available serotonin measuring reagent "Serotonin EIA" (prepared by Labor Diagnostika Nord, corporation). Relative serotonin concentrations of control section 2 and test section were obtained in relation to control section 1 when the serotonin concentration in brain of control section 1 is set to 100, and the results were shown in Table 3.
[Table 3]

| | Near ultraviolet radiation | | Relative aggressiveness (%) | Relative serotonin n concentration in brain (%) |
|---|---|---|---|---|
| | in bright period | in dark period | | |
| Control section 1 | not radiated | not radiated | 100 | 100 |
| Control section 2 | not radiated | radiated | 103.6 | 94.5 |
| Test section | radiated | not radiated | 55.2 | 139.4 |

In the mice of control section 2 where the near ultraviolet radiation was emitted in the dark period only, aggressiveness and serotonin concentration in brain showed no difference as compared with control section 1. On the other hand, in the mice of test section bred under the illumination of the present invention, aggressiveness decreased in comparison with control section 1 and serotonin concentration in brain was high. From the above results, it is understood that the lighting device of the present invention has the advantageous effects of activating animal's serotonin nervous system to decrease aggressiveness, and such advantageous effects are not obtained either visible light or near ultraviolet radiation only, but are obtained by the radiation of light which contains both of visible light and near ultraviolet radiation.

Industrial Applicability

As explained above, the lighting device of the present invention can be used as illumination at nighttime in daily life space, activates serotonin nervous system, and does not raise noradrenaline level in blood substantially, and through these functions the lighting device of the present invention has advantageous effects of suppressing aggressiveness of animals including human beings. According to the present invention, the factors inducing emotional and aggressive so-called "kireru" state are reduced through the use of the present invention, and abnormal behaviors and crimes are suppressed. This is profit expected from the present invention. In this point, the present invention is useful to the society.

**Claims**

1. A lighting device, which radiates light that activates human serotonin nervous system.

2. A lighting device according to claim 1, which radiates light that does not substantially raise noradrenaline level in human blood.

3. A lighting device according to claim 1 or 2, wherein the light being radiated contains near ultraviolet radiation having a wavelength of 320 nm or longer but shorter than 380 nm along with visible light.

4. A lighting device according to claim 3, wherein the radiant energy of the near ultraviolet radiation is in the range of 3 to 15 % of that of the visible light.

5. A lighting device according to any one of clams 1 to 4, wherein the light being radiated is white light in the nature of light bulb color or daylight color (color temperature ranging from 2700 to 6500) and has 95 or more general color rendering index (Ra).

6. A lighting device according to any one of claims 1 to 5, wherein the source of the light is one selected from light emitting diode (LED) and organic electroluminescent device (organic EL).

7. A lighting device according to any one of claims 1 to 6, which comprises a light source radiating visible light, a light source radiating near ultraviolet radiation having a wavelength in the range of 320 to 380 nm, a power source circuit for supplying electric power to the light sources, and a lightning circuit for lightening the light sources, and a control circuit.

Figure 1:

Figure 2:

Figure 3:

<div style="text-align: center">**INTERNATIONAL SEARCH REPORT**</div>

| International application No. |
| --- |
| PCT/JP2009/067573 |

A.  CLASSIFICATION OF SUBJECT MATTER
*F21S2/00*(2006.01)i, *A61M21/02*(2006.01)i, *H01L51/50*(2006.01)i, *F21Y101/02*
(2006.01)n, *F21Y105/00*(2006.01)n, *H01L33/00*(2010.01)n

According to International Patent Classification (IPC) or to both national classification and IPC

B.  FIELDS SEARCHED

Minimum documentation searched (classification system followed by classification symbols)
F21S2/00, A61M21/02, H01L51/50, F21Y101/02, F21Y105/00, H01L33/00

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X<br>Y<br>A | Hideho ARITA, Ohisama Therapy Serotonin<br>Seikatsu no Susume, Seishun Publishing Co.,<br>Ltd., 01 June 2006 (01.06.2006), pages 92, 93 | 1<br>3,5-7<br>2,4 |
| Y | JP 2005-177149 A (Matsushita Electric Works,<br>Ltd.),<br>07 July 2005 (07.07.2005),<br>paragraphs [0054] to [0058]; fig. 6<br>(Family: none) | 3,5-7 |
| Y | JP 2008-218485 A (Toshiba Lighting & Technology<br>Corp.),<br>18 September 2008 (18.09.2008),<br>paragraphs [0053], [0057], [0059]<br>& CN 101257012 A | 5 |

☐  Further documents are listed in the continuation of Box C.          ☐  See patent family annex.

| | |
| --- | --- |
| *      Special categories of cited documents:<br>"A"   document defining the general state of the art which is not considered<br>         to be of particular relevance<br>"E"   earlier application or patent but published on or after the international<br>         filing date<br>"L"    document which may throw doubts on priority claim(s) or which is<br>         cited to establish the publication date of another citation or other<br>         special reason (as specified)<br>"O"   document referring to an oral disclosure, use, exhibition or other means<br>"P"    document published prior to the international filing date but later than<br>         the priority date claimed | "T"    later document published after the international filing date or priority<br>         date and not in conflict with the application but cited to understand<br>         the principle or theory underlying the invention<br>"X"    document of particular relevance; the claimed invention cannot be<br>         considered novel or cannot be considered to involve an inventive<br>         step when the document is taken alone<br>"Y"    document of particular relevance; the claimed invention cannot be<br>         considered to involve an inventive step when the document is<br>         combined with one or more other such documents, such combination<br>         being obvious to a person skilled in the art<br>"&"    document member of the same patent family |
| Date of the actual completion of the international search<br>       25 December, 2009 (25.12.09) | Date of mailing of the international search report<br>       12 January, 2010 (12.01.10) |
| Name and mailing address of the ISA/<br>       Japanese Patent Office | Authorized officer |
| Facsimile No. | Telephone No. |

Form PCT/ISA/210 (second sheet) (April 2007)

<div style="text-align: center">11</div>

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP KOKAI2005247976 A

**Non-patent literature cited in the description**

• **Hideho ARITA.** Brain deficient in serotonin. NHK SHUPPAN, 2003

• **Randy J. Nelson ; Brian C. Trainor.** Neural mechanism of aggression. *Nature reviews/Neuroscience,* 2007, vol. 8, 536-546